**Europäisches Patentamt**

(19) **European Patent Office**

**Office européen des brevets**

(11) Publication number: **0 149 539**
**A2**

(12) # EUROPEAN PATENT APPLICATION

(21) Application number: **85300202.0**

(22) Date of filing: **11.01.85**

(51) Int. Cl.⁴: **C 12 P 13/22**
**C 12 P 13/04, C 12 N 15/00**
**C 12 N 1/20**
**//(C12N1/20, C12R1:19)**

(30) Priority: **13.01.84 CA 445228**

(43) Date of publication of application:
**24.07.85 Bulletin 85/30**

(84) Designated Contracting States:
**DE FR GB IT SE**

(71) Applicant: **STAUFFER CHEMICAL COMPANY**

**Westport Connecticut 06881(US)**

(72) Inventor: **Newman, Elaine B.**
**1843 Tupper Avenue**
**Montreal Quebec, H3H 1N3(CA)**

(74) Representative: **Smith, Sydney et al,**
**Elkington and Fife High Holborn House 52/54 High**
**Holborn**
**London WC1V 6SH(GB)**

(54) **Mutant strains deficient in L-serine deaminase activity and use thereof.**

(57) A mutant strain of bacteria characterised in that it is deficient in L-serine deaminase activity is disclosed.

Such may be used in the production of amino acids or precursors thereof and such a process is also disclosed.

The invention offers advantages.

EP 0 149 539 A2

Croydon Printing Company Ltd.

"Mutant strains deficient in L-serine deaminase activity and use thereof"

The invention relates to a mutant bacterial strain deficient in the activity of 2-serine hydro-lyase (deaminating), hereinafter referred to as L-serine deaminase, an enzyme substantially similar in activity to one isolated from Clostridium acids-urici and identified as EC 4.2.1.14 (hereinafter referred to as "L-serine deaminase activity"). (See Carter, J.F., and Sayers, R.D., "Clostridium acids-urici (EC 4.2.1.14)", J. Bacteriol. 109:757-763 (1972). In particular, the invention relates to several mutants of the microorganisms Escherichia coli (E. coli) K12 all of which are deficient in L-serine deaminase (L-SD) activity.

The production of L-tryptophan from carbohydrates in microorganisms has been accomplished in the prior art essentially by two approaches. The first of these approaches has been to select mutants of a wild-type microorganism that are feedback resistant to tryptophan and its analogs. Examples of this approach include a strain of Bacillus subtilis resistant to 5-fluorotryptophan (U.S. Patent 4,363,875); Brevibacterium species resistant to 5-methyltryptophan (U.S. Patent 3,700,539); and Corynebacterium glutamicus ATCC 21851 which requires phenylalanine and tyrosine and is resistant to 4-methyltryptophan, 6-fluorotryptophan, 4-aminophenylalanine, 4-fluorotryptophan, tyrosine-hydroxamate and phenylalanine hydroxamate. The latter strain is capable of 16.8 mg/l tryptophan production from 15 g/dl sugar derived from cane blackstrop molasses.

A second approach to increasing tryptophan production has been to supply a host microorganism with genetic information, carried on a plasmid or plasmids, to control tryptophan biosynthesis. An example of the

0149539

latter method is U.S. Patent 4,371,614 which describes an E. coli host deficient in the enzyme tryptophanase [EC 4.1.99.1] transformed by plasmids carrying information for tryptophan synthesis.

Whether the microorganism used for tryptophan synthesis is a mutant strain or transformed strain, the synthesis of tryptophan proceeds along the following pathway.

CHART 1

Sugar

(at least 15 steps)

chorismic acid

Anthranilic acid synthetase

Anthranilic acid → N-(5'-phosphoribosyl)-anthranilic acid → Enol-1-(o-carboxyphenyl amino)-1-deoxyribulose-5-phosphate → Indole-3-glycerolphosphate

Prephenic acid → p-Hydroxyphenyl pyruvic acid → Tyrosine

Prephenic acid → Phenylpyruvic acid → Phenylalanine

+ serine

L-tryptophan

Tryptophanase

Breakdown products (Indole)

3.

0149539

Anthranilic acid is the first major precursor in the tryptophan biosynthetic pathway. The last step of the pathway for the production of tryptophan is the tryptophan synthase- mediated condensation of a molecule of indole-3-glycerol- phosphate and a molecule of serine to form tryptophan and glyceraldehyde-3-phosphate.

It has been observed that in microorganisms in which the pools of tryptophan precursors up through indole-3- glycerolphosphate are plentiful, the supply of serine determines the rate of tryptophan production. Serine biosynthesis generally proceeds along the following pathway.

CHART 2

3-phosphoglyceric acid

↓

3-phosphohydroxypyruvic acid

↓

3-phosphoserine

↓

serine  ————→ glycine  (folate)

              L-serine dehydratase

↓           (L-serine deaminase)

pyruvate

↓

acetyl CoA

Serine is depleted in a number of pathways. In one, through the action of tetrahydrofolic acid, serine is converted to glycine. In another major degradation pathway L-SD acts on serine, leading to the production of pyruvate and acetyl CoA. In <u>Clostridium acids-urici</u>, L-SD is believed to be one of the enzymes of the uric acid fermentation pathway and in general appears to play a role in a number of pathways in which serine is generated and further metabolized as part of the main carbon pathway. The level of L-SD varies as a function of nitrogen nutrition, carbon source and amino acids availability. Inducers of L-SD activity are well characterized. These include absence of nitrogen, presence of glycine and leucine (but not serine, although serine does not affect induction by glycine and leucine) and growth in minimal glucose medium.

Thus in glucose medium, the most economical growth medium for the production of amino acids, L-SD levels are high (Newman, E.B. and Kapoor, V. "In vitro studies on L-serine deaminase activity of Escherichia coli K12", Can. J. Biochem. 58, 1292-1297 (1980)), causing, it is believed, a decrease in the cellular levels of serine by deamination, maintaining the serine pool at low levels and avoiding the inhibitory effect of L-serine on L-threonine deaminase (Isenberg, S. and Newman, E.B., "Studies on L-serine deaminase in Escherichia coli K12", J. Bacteriol. 118, 53-58 (1974)). What is certain is that in the presence of L-SD, E. coli K12 converts L-serine irreversibly to pyruvate or to a compound at the same oxidation level as pyruvate, indicating that even if L-serine is not the natural substrate of L-SD, it can be degraded by L-SD in a physiologically significant reaction (Newman, E.B. and Walker, C., "L-serine degradation in Escherichia coli K12: a combination of L-serine, glycine and leucine used as a source of carbon", J. Bacteriol. 151: 777-782 (1982)).

The availability of serine for tryptophan biosynthesis or for the fermentative production of serine can be enhanced by utilizing microorganisms that are deficient in enzymes that degrade serine. Microorganisms deficient in folate are generally too metabolically stressed to be maintained in culture. However, microorganisms deficient in L-SD can be maintained in culture and provide a source for enhanced tryptophan biosynthesis.

The usefulness of microorganisms deficient in L-SD activity in tryptophan production is particularly apparent in microorganisms that have been altered for augmented tryptophan production. For example, microorganisms such as those disclosed in U.S. Patent 4,371,614 in which an E. coli K12 strain deficient in the enzyme tryptophanase has been transformed by plasmids carrying genetic information for tryptophan biosynthesis, have been shown to produce tryptophan in amounts greater than that produced by E. coli strains lacking these alterations. Among the limitations to the production of tryptophan in these strains is the degradation of L-serine, whether synthesized by the microorganisms or supplied exogenously$_1$ by L-SD.

Tryptophan production in strains such as those described in U.S. Patent 4,371,164 and other strains modified for increased tryptophan production may be further augmented by growing phage $P_1$ on an L-SD deficient strain, transducing the tryptophan-producing strain with phage $P_1$ and screening transductants for reduced L-SD activity or increased tryptophan production or both.

The inventor has made and isolated a number of strains of microorganisms, particularly gram-negative microorganisms such as E. coli, that are deficient in or lack the activity of L-SD. The term "L-SD deficient" as used herein means having an L-SD activity (as determined by the method described in the examples hereinbelow) less than the wild type parental strain. According to this definition strains lacking L-SD activity display L-SD activities in a range of 5 milliunits or less (as defined in the examples hereinbelow). E. coli strains deficient or lacking in L-SD activity are unable to grow on serine, glycine- and leucine-containing medium and lack L-SD activity when grown in glucose minimal medium. The E. coli strains according to the invention were isolated after exposing a strain of E. coli K12 to ultraviolet radiation (UV) or transposon mutagenesis utilizing Mu dX insertions in E. coli K12 and screening for L-SD deficient mutants on an appropriate medium.

Procedures described according to the invention provide a means for making and isolating from widely available microorganisms, including gram-negative microorganisms such as E. coli, mutant strains that are deficient in L-SD activity.

Figure I is a diagram showing the relationship between various amino acid concentrations and the yield of strain 181-34.

Figure II is a schematic restriction map of plasmid pD2643.

The invention will be better understood from the following examples:

## EXAMPLE 1

A. Isolation of Strain MEW 128

Strain CU1008, a wild-type E. coli K12 carrying a deletion in ilvA was obtained from L.S. Williams Washington University, School of Medicine, St. Louis, Missouri, USA.

Strain MEW 128: Strain CU1008 was irradiated with UV light, subcultured in glucose minimal medium and incubated with penicillin in medium supplemented with serine 2 mg/ml, glycine 200 μg/ml and leucine 50 μg/ml, at 37°C, according to the method of Davis, B.D. et al., "The isolation of biochemically deficient mutants of bacteria by means of penicillin", J. Amer. Chem. Soc. 70:4267 (1948). Survivors were plated on glucose minimal medium and screened by replicate plating on

0149539

medium made with Bacto-Agar (Trademark) supplemented with serine, glycine and leucine as above (SGL-plates). Strain MEW 128 was selected as unable to grow on SGL-plates, and also proved unable to grow in liquid glucose minimal medium. This strain is also thiamine-requiring. It has been deposited in the American Type Culture Collection (ATCC), 12301 Parklawn Drive, Rockville, Maryland, U.S.A. 20852, under accession number 39577 on January 12, 1984.

Although the initial isolation of strain MEW 128 was carried out as described above, strain MEW 128 is consistently selected by replicate plate screening on glucose minimal media supplemented with serine, glycine and leucine as above, solidified with 8 g/l Gelrite (Trademark-Kelco 63002A) and further including thiamine 10 mg/l, ferric chloride 5 mg/l, sodium molybdate 0.1 mg/l and manganese chloride 0.12 mg/l. Strain MEW 128 did not grow on this medium.

B. Enzyme Assays

L-SD activity was assayed in toluene-treated whole cells as described in Isenberg, S. and Newman, E.B., "Studies on L-serine deaminase in <u>Escherichia coli</u> K12", J. Bacteriol. 118:53-58 (1974), which is herein incorporated by reference. Results are found in Table 1 below and are expressed in milliunits (mu), i.e. nanomoles keto acid produced by 0.1 ml of a 100 Klett units suspension of cells in 35 minutes. Five mu is the lowest reliable value in this assay.

## TABLE I

### L-SD Activity In Strain MEW 128 and Related Strains

| Expt. No. | Incubation Conditions | CU1008 | MEW 128 | Thiamine-Independent Revertant |
|-----------|----------------------|--------|---------|-------------------------------|
| 1. | glucose minimal medium | 19 | 5 | 7 |
| 2. | glycine and leucine | 110 | 6 | 31 |
| 3. | UV-irradiated | 130 | 5 | 38 |
| 4. | 42°C | 34 | 5 | 25 |
| 5. | LB medium * | 230 | 30 | 51 |

\* LB medium contains 10 g/l Bactotryptone (Trademark), 5 g/l Bactoyeast Extract (Trademark) and 10 g/l NaCl (Miller, J.H., "Experiments in Molecular Genetics", Cold Spring Harbour (1973), hereinafter referred to as Miller).

Exponential phase cells growing in glucose minimal medium at 37°C were subcultured in five conditions: in the same medium at 37°C (Expt. 1) and at 42°C (Expt. 4), in the same medium with amino acids (Expt. 2), and in LB medium (Expt. 5). Some of the cells were irradiated with ultraviolet light for 60 seconds and then incubated in glucose minimal medium (Expt. 3). Incubations were at 37°C unless otherwise stated. L-SD activity was determined one hour after the subculture. Details of the experimental methods are as described in Newman, E.B. et al., "L-serine deaminase activity is induced by exposure of Escherichia coli K12 to DNA-damaging agents", J. Bacteriol., 152: 702-705 (1982).

### C. Characterization of MEW 128

#### 1. Growth Requirements

Strain MEW 128 is unable to grow on glucose minimal medium plates supplemented with serine, glycine and leucine at the concentrations described above. Glucose minimal medium plates support growth of MEW 128, but liquid glucose minimal medium does not unless supplemented with 1/200 (vol/vol) LB medium. The requirement for LB medium can be replaced by 3 µg/l thiamine. The thiamine requirement can be by-passed by supplementing liquid glucose minimal medium with concentrations of succinate and acetate exceeding 300 µg/ml each or with 5 µg/ml lysine and 10 µg/ml methionine. True thiamine-dependent strains lack a functional lactic acid dehydrogenase (LDH) as this enzyme requires thiamine as a co-factor. Thus, thiamine-dependent strains of microorganisms excrete pyruvate. The keto acid excreted by MEW 128 was identified by reacting non-acidified culture supernatant with commercial LDH (Boehringer Mannheim Co) and assaying for NADH oxidation (Hohorst, H.-J., "L-(+) lactate determination with LDH and TPN", Methods of Enzymatic Analysis, Ed. H.V. Bergmyer, Verlag Chemie, Academic Press, 2d Edition, 1965). The results showed that substantially all of the keto acid was pyruvate.

2.  L-SD Activity

Strain MEW 128 grown at 37°C in glucose minimal medium with LB medium 1/50 showed little (5 mu) or no L-SD activity (Table I). However, growth in LB medium induced a substantial amount of activity (30 mu) though much less than in the parent strain (230 mu) grown in the same conditions. The other known inducers of L-SD activity - i.e. UV, glycine and leucine, and increased temperature (42°C) - had no significant effect (all about 5 mu). It is clear that strain MEW 128 retained the ability to produce some L-SD activity, though it did not respond to most of the usual inducing conditions.

The same experiment was repeated with cells grown in glucose minimal medium with thiamine with the same results. The addition of excess thiamine thus did not restore L-SD activity. To be sure that thiamine does not normally affect L-SD activity, L-SD was assayed as described above in cells of the parent strain CU1008 grown with and without thiamine. No difference in activity could be seen (data not shown).

In summary, strain MEW 128 differed from its parent strain in two ways: it required thiamine for growth, and it was deficient in L-SD activity, even under L-SD inducing conditions.

3. Thiamine-Independent Revertants of MEW 128

Revertants of MEW 128 able to grow in glucose minimal medium without thiamine were isolated. Single-colony isolates of strain MEW 128 were inoculated into liquid glucose minimal medium and incubated until well grown. Single colonies were isolated from the liquid cultures by plating on glucose minimal medium plates. Since strain MEW 128 grows on glucose minimal medium plates, it is clear that only the liquid medium selects for thiamine independence.

Twenty-five spontaneously isolated thiamine-independent strains were assayed for L-SD activity in glucose minimal medium using the above-described method and all showed some activity. Five of these were assayed for L-SD activity in glucose minimal medium and in medium with 250 $\mu$g/ml glycine and 50 $\mu$g/ml leucine. L-SD values varied from 9 to 18 mu uninduced and from 27 to 47 mu induced. Restoration to wild-type values was never seen. One revertant shown in Table I was assayed in all inducing conditions. Only partial induction was observed for all conditions.

EXAMPLE II

A. Isolation of Strain MEW 191

A derivative of strain CU1008 carrying a deletion of the lac operon (hereinafter CU1008 $\Delta$ lac) was constructed by isolating a proline-requiring auxotroph by penicillin selection and transducing to proline independence with a donor carrying a deletion of the lac operon. The donor strain, CAG5050 (Mu dX CAM (Ap$^r$ lac) pro lac his met tyr rpsL nal$^r$/F' pro lac Z$_{8305}$::Mu c$^{ts}$62), was obtained from M. Howe, University of Wisconsin, Madison, Wisconsin, U.S.A. This strain is lysogenic for Mu dl B$^x$::Tn9, an X mutant of the Mu dl phage (Casadaban, M.J. and Cohen, S.N., "Lactose genes fused to exogenous promoters in one step using a Mu-lac bacteriophage: in vivo probe for transcriptional control sequences", Proc. Natl. Acad. Sci. U.S.A. 76: 4530-4533 (1979)), derived by Tn9 insertion into the B gene (see Baker, T.A. et al., "Mu dX, a derivative of Mu dl (lac Ap$^r$) which makes stable lacZ fusions at high temperatures", J. Bacteriol. 156: 970-974 (1983)).

Strain CU1008 Δ lac was transduced to chloramphenicol and ampicillin resistance with phage Mu dX, which makes stable lacZ fusions at high temperature. The transduced strain, designated MEW 191, was isolated as an ampicillin- and chloramphenicol-resistant strain unable to grow on SGL-plates. This strain has been deposited with the ATCC under accession number 39575 on January 12, 1984.

B. Enzyme Methods

The same methods used in Example I were used in Example II.

C. Characterization of Strain MEW 191

Growth requirements: Strain MEW 191 was chloramphenicol- and ampicillin-resistant, unable to grow with serine, glycine and leucine and showed the same phenotype as strain MEW 128: requirement for thiamine, no L-SD activity in glucose minimal medium, and altered response to inducers (Table II). L-SD activity in strain MEW 191 was substantially similar to the L-SD activity of strain MEW 128. This strain also excreted pyruvate in the absence of thiamine.

### TABLE II

### L-SD Activity in Strain MEW 191 and Related Strains

| Incubation | MEW 191 | Revertants | | |
|---|---|---|---|---|
| | | 1 | 2 | 3 |
| glucose minimal medium | 5 | 23 | 15 | 12 |
| glycine and leucine | 5 | 101 | 50 | 46 |
| UV-irradiated | 5 | 103 | nd[a] | nd |
| 42°C | 5 | 37 | nd | nd |
| LB medium | 12 | 173 | nd | nd |

[a]nd = not determined. Experiment done as in Table I.

### D. Thiamine-Independent Revertants of MEW 191

Revertants of strain MEW 191 able to grow with serine, glycine and leucine were then isolated. Some of these were resistant to chloramphenicol and ampicillin, but several were not. Four antibiotic-sensitive colonies were tested further. They proved to be thiamine-independent, and contained L-SD activity at a level similar to that of the parent strain. Higher levels were induced by growth with glycine and leucine, though not as high as in strain CU1008.

### EXAMPLE III

### A. Isolation of Strain IK 15-5 - An L-SD Deficient Thiamine- Non-Requiring Strain

The same methods and the same parental strain, CU1008 $\Delta$ lac, used in Example II were used in Example III to isolate strain IK 15 which did not grow on SGL-plates. Employing conventional phage transduction methods (see Miller), phage $P_1$ grown on strain IK 15 was used to transduce strain CU1008 $\Delta$ lac to ampicillin and chloramphenicol resistance. Strain IK 15-5 was isolated as a transductant of CU1008 $\Delta$ lac

devoid of L-SD activity, resistant to ampicillin and chloramphenicol and unable to grow on SGL-plates. This strain has been deposited with the ATCC under accession number 39576 on January 12, 1984.

B.  Enzyme Methods

The same methods used in Example I were used in Example III.

C.  Characterization of Strain IK 15-5

1.  Growth Requirements

Strain IK 15-5 was chloramphenicol- and ampicillin- resistant and unable to grow on SGL-plates. Significantly, IK 15-5 had no requirement for thiamine.

2.  L-SD Activity

IK 15-5 had no L-SD activity in glucose minimal medium and altered L-SD activity under inducing conditions as indicated in Table III. L-SD activity in strain IK 15-5 was substantially similar to the L-SD activity of strain MEW 128. Growth and assay conditions were the same as those used in Example I, Table I.

TABLE III

| Incubation Conditions | L-SD Activity in Strain IK 15-5 (mu) |
|---|---|
| glucose minimal medium | 2 |
| glycine and leucine | 9 |
| UV irradiation | 3 |
| 42°C | 4 |
| LB medium | 75 |

## EXAMPLE IV
### Characterization of Genetic Defects in Strains
### Showing Reduced L-SD Activity

A.  Defects in the Structural Gene for L-SD

Studies of transcription of $\beta$-galactosidase from the insertion in the gene mutated in strain IK 15-5, using the methods described in Miller, indicate that the insertion occurs in a structural gene for L-SD.  As shown in Table IV, $\beta$-galactosiuase transcription is increased under conditions otherwise known to induce L-SD, although no significant increase in L-SD activity is observed in this strain as compared to the parental strain.  Growth and assay conditions are the same as in Example III.

### TABLE IV

| L-SD Incubation Conditions | Strain IK 15-5 | |
| --- | --- | --- |
| | $\beta$-Galactosidase Activity (Units/mg protein) | L-SD Activity (mu) |
| glucose minimal medium | 6u | 2 |
| glycine and leucine | 122 | 9 |
| UV irradiation | 99 | 3 |
| 42°C | 57 | 4 |
| LB medium | 225 | 75 |

Transcription of $\beta$-galactosidase from the insertion in the gene mutated in strain IK 15-5 is increased by a mutation that is known to increase L-SD activity in wild type E. coli K12 strains.

The ssd mutation in wild type E. coli K12 strains is pleiotrophic.  Among its effects is a marked increase in the expression of L-SD activity and the inability to use succinate as carbon source as described in Morris, J.F. and Newman, E.B., "Map location of the ssd mutation in Escherichia coli K12", J. Bacteriol. 143: 1504-1505 (1980).  Methods for the isolation of ssd

0149539

mutants are described in Newman, E.B., et al., "L-serine degradation in _Escherichia coli_ K12: directly isolated _ssd_ mutants and their intragenic revertants", J. Bacteriol. 150: 710-715 (1982).

The _ssd_ mutations in CU1008 are isolated by their ability to grow on medium containing 2 mg/ml L-serine. The _ssd_ mutation is transduced into a CU1008 _metB_$^-$ recipient by phage $P_1$ transduction using conventional techniques and selecting the transductants for methionine independence and screening for colonies able to grow at the expense of L-serine. Such a strain, designated CU1008 _ssd_ is then transduced with phage $P_1$ grown on strain IK 15-5. Chloramphenicol- and ampicillin-resistant colonies unable to grow on serine, glycine and leucine and independent for methionine are isolated. A strain with this phenotype is additionally unable to use succinate as carbon source and is designated as IK/_ssd_.

Strain IK/_ssd_ was assayed for $\beta$-galactosidase activity as previously described and had 3 fold more $\beta$-galactosidase activity than IK 15-5, but no significant increase in L-SD activity. The results comparing $\beta$-galactosidase activity and L-SD activity for strain IK/_ssd_ and IK 15-5 are shown in Table V and indicate that _ssd_ effects an increase in transcription of the $\beta$-galactosidase sequence residing in the L-SD structural gene of IK 15 derivatives.

### TABLE V

| | Strain IK/ssd | | Strain IK 15-5 | |
|---|---|---|---|---|
| L-SD Inducing Conditions | $\beta$-Galacto-sidase Activity (units/mg protein) | L-SD Activity (mu) | $\beta$-Galacto-sidase Activity (units/mg protein) | L-SD Activity (mu) |
| glucose minimal medium | 179 | 1 | 60 | 2 |
| glycine and leucine | 425 | 4 | 122 | 9 |
| LB medium | 645 | 53 | 225 | 75 |

B.  Defect in a Regulatory Gene of Strain MEW 128
    Affecting Transcription of the L-SD Structural Gene

A double mutant containing the mutations found in MEW 128 and the mutation found in strain IK 15 was isolated as follows:  using conventional techniques phage $P_1$ grown on strain IK 15 was used to transduce an Mu lysogen of strain CU1008 to chloramphenicol- and ampicillin-resistance.  An Mu lysogenic strain resistant to these antibiotics and L-SD deficient as determined by its inability to grow on SGL-plates was isolated and was designated IK 15-3.  Using conventional techniques, phage $P_1$ grown on strain IK 15-3 was used to transduce strain MEW 128.  A strain designated 128/IK 15-3 having antibiotic resistance, L-SD deficiency as determined by its inability to grow on SGL-plates and requiring thiamine for growth was isolated.

Strain 128/IK 15-3 was assayed for $\beta$-galactosidase activity and L-SD activity under L-SD induction conditions using methods described previously.  IK 15-3 was assayed under identical conditions.  The results shown in Table VI indicate that transcription of $\beta$-galactosidase from the IK 15-3 promotor is inhibited in strains also carrying the MEW 128 mutation and that the MEW 128 mutation occurs in a regulatory gene that prevents the transcription of the structural gene for L-SD.

## TABLE VI

| L-SD Incubation Conditions | $\beta$-Galactosidase Activity (Units/mg protein) | | L-SD Activity (mu) | |
|---|---|---|---|---|
| | IK 15-3 | 128/IK 15-3 | IK 15-3 | 128/IK 15-3 |
| glucose minimal medium | 44 | non-detectable | 2 | 1 |
| glycine and leucine | 150 | non-detectable | 13 | 4 |
| LB medium | 225 | non-detectable | 75 | 50 |

## EXAMPLE V

## Reduced Degradation of Serine by E. Coli Strains Deficient in L-SD Activity

A. Effects of Limited Serine Availability on Growth of L-SD Deficient E. Coli Strains Auxotrophic for Serine and/or Glycine

Three auxotrophs requiring serine or glycine for growth on glucose minimal medium were isolated from strain CU1008 Δ lac. Two of the auxotrophs were isolated by insertion mutagensis using Mu dX as described in Example II. The auxotrophs were screened for serine requirement. Two strains designated DR1 and DR4 were isolated. A third auxotroph designatged 181-34 was constructed from CU1008 Δ lac using as donor a lysate of E. coli strain Mal 103: $F^-$, Mu dl ($Ap^r$ lac) ara B::Mu c ts, ara D139, Δ (lac IPOZYA) U169, strA. The strain was obtained from E. McFall, New York University, School of Medicine, New York, New York, USA. Strain 181-34 was isolated by selecting for ampicillin-resistance and screening for serine requirement.

Strain MEW 128 was transduced to antibiotic-resistance using conventional techniques, with phage $P_1$ grown on strains DR1, DR4 and 181-34. Double mutants which were antibiotic-resistant serine and/or glycine auxotrophs unable to grow with serine, glycine and leucine as carbon source were identified and designated as 128/DR1, 128/DR4 and 128/181-34.

The three auxotrophs DR1, DR4 and 181-34 and three double mutants 128/DR1, 128/DR4 and 128/181-34 were grown in glucose (2 mg/ml) minimal medium supplemented with 300 μg/ml L-serine, which is an amount of serine previously determined to be insufficient to supply all serine required for growth of the serine auxotroph. Lightly inoculated cultures of the six strains were grown at 37°C in this medium to the stationary phase as determined by turbidity in a colorimeter and the final yield of each strain was determined as measured by mg protein /ml culture medium (Lowry determination). L-SD activity was also determined for each strain using the method previously described. As shown in Table VII, in all cases the double mutants which are serine/glycine auxotrophs deficient in L-SD activity, achieved higher yields than the corresponding serine/glycine auxotrophs.

## TABLE VII

| Strain | L-SD Activity (mu) | Yield (ug protein/ml culture) |
|---|---|---|
| DR1 | 90 | 30 |
| 128/DR1 | 6 | 38 |
| DR4 | not determined | 31 |
| 128/DR4 | 6 | 40 |
| 181-34 | 96 | 27 |
| 128/181-34 | 5 | 46 |

B. Effect of Limited Serine Availability on Growth of E. coli Strains Auxotrophic for Serine/Glycine under L-SD Induction Conditions

Strain 181-34 which is auxotrophic for serine/glycine but is not L-SD deficient was grown with limited serine (300 $\mu$g/ml medium) in conditions previously determined to be L-SD inducing and the yield ($\mu$g protein/ml medium) of 181-34 was determined at stationary phase. Low yields were observed as is shown in Table VIII.

## TABLE VIII

| Growth Conditions | Yield ($\mu$g protein/ml culture) |
|---|---|
| glucose minimal medium plus 300 $\mu$g/ml L-serine at 37°C | 27 |
| glucose minimal medium plus 300 $\mu$g/ml serine at 37°C plus 50 $\mu$g/ml leucine | 16 |
| glucose minimal medium plus 300 $\mu$g/ml serine at 42°C | 19 |

C. Effect of Increasing Exogenous Serine Concentration
on Yield in an E. coli Strain Auxotrophic for
Serine/Glycine

Aliquots of strain 181-34 were lightly inoculated into glucose minimal medium and cultured at 37°C with increasing concentrations of exogenous serine and the yield was determined at stationary phase as described previously. For comparison, the same strain was grown under the same conditions except that increasing concentrations of glycine were added. The results shown in Table IX and Figure I show that final growth of the strain is not linear with the initial concentration of either serine or glycine. However, a plateau in yield is reached at above about 200 $\mu$g/ml glycine, but no corresponding yield plateau is reached at any concentration of serine tested. The absence of a yield plateau for serine shows that the amount of initial serine remains growth-limiting even at high concentrations, indicating that serine is depleted to growth-limiting concentrations during growth of an L-SD producing strain of E. coli.

TABLE IX

Strain 181-34

| Concentration of Amino Acid Supplied (ug/ml) | Yield (mg protein/ml medium with serine) | Yield (mg protein/ml medium with glycine) |
|---|---|---|
| 0 | 0 | 0 |
| 50 | 40 | 140 |
| 100 | 70 | 192 |
| 200 | 135 | 250 |
| 250 | 166 | 260 |
| 300 | 179 | 280 |
| 400 | 211 | 250 |
| 500 | 230 | 260 |
| 600 | 245 | 260 |
| 900 | 290 | 280 |
| 1000 | 300 | not determined |

## EXAMPLE VIII

Utilization of L-SD Deficient E. coli Strains
in the Construction of a Tryptophane Over-Producing
Strain

### A. Construction of Strain C534

B1238 is an E. coli K12 strain (obtained from A. Campbell's Laboratory, Stanford University, Palo Alto, California, USA) and is $Gal^-Bio^-Trp^-$. Strain B1238GB was a $Gal^+Bio^+P_1$ transductant of B1238 (donor strain: W3110) and strain A103 was an anthranilate-resistant derivative of B1238GB selected after EMS mutagenesis. Strain C534 was derived from A103 by transduction of $Sal^+Tna^-$ (tryptophanase-negative) using $P_1$ grown on donor strain C537.

### B. Construction of Strain C534SD

$P_1$ phage grown on L-SD-negative strain MEW 191 may be used to transduce C534 to $Ap^rCm^r$. One transductant is named C534SD. Unlike strain C534, strain C534SD lacks L-SD activity in glucose minimal medium and is thiamine-requiring.

### C. Construction of Plasmid-Bearing Derivatives of C534 and C534SD

Plasmid pD2643 is a derivative of pBR322 carrying the trpA, trpB, trpC and trpD genes of E. coli and specifying resistance to tetracycline (See Figure II). This plasmid was introduced into strains C534 and may be introduced into C534SD by transformation to $Tc^r$ as previously described. C534(pD2643) and C534SD(pD2643) are representative transformants.

### D. Fermentation of Anthranilate to Tryptophan by Strains C534(pD2643) and C534SD(pD2643)

These strains are grown in 20ml Vogel-Bonner minimal medium supplemented with 2.0% dextrose, 0.4%

NZ-amine, thiamine (1 μg/ml), tetracycline (20 μg/ml) and anthranilic acid (1000 μg/ml) in 250 ml shake flasks on a rotary shaker (280 rpm) at 30°C.

Samples are periodically removed from the shake flasks and are assayed for tryptophan and indole concentrations against authentic tryptophan and indole standards.  The tryptophan yield of C534SD(pD2643) consistently exceeds that of C534(pD2643).

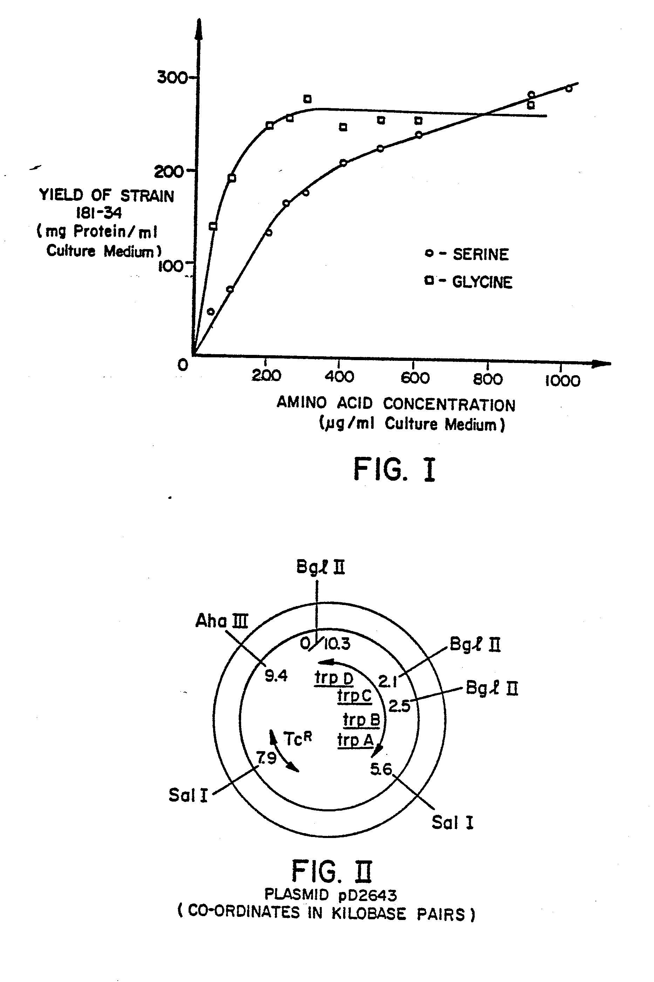

Claims:

1. A mutant strain of bacteria characterised in that it is deficient in L-serine deaminase activity.

2. A mutant strain as claimed in claim 1 wherein there is a mutation in a structural gene and/or a regulatory gene for L-serine deaminase activity.

3. A mutant strain as claimed in claim 2 wherein the mutation is an insertion mutation.

4. A mutant strain as claimed in claim 3 wherein the insertion is a Mu dX CAM(Ap$^r$ lac) insertion.

5. A mutant strain as claimed in any of claims 1 to 4 wherein the bacteria are of the species escherichia coli.

6. A mutant strain as claimed in claim 5 wherein the E. coli is strain K 12.

7. A mutant strain as claimed in any of claims 1 to 6 wherein it requires thiamine.

8. A mutant strain as claimed in claim 1 wherein it is E. coli MEW 128 (ATCC 39577) or E. coli MEW 191 (ATCC 39575) or E. coli IK 15-5 (ATCC 39576).

9. A mutant strain as claimed in claim 8 wherein it exhibits similar L-serine deaminase activity characteristics.

10. A process for the production of an amino acid or a precursor thereof characterised in that a mutant strain as claimed in any of claims 1 to 9 is used.

**FIG. I**

**FIG. II**

PLASMID pD2643

( CO-ORDINATES IN KILOBASE PAIRS )